Europäisches Patentamt

European Patent Office

Office européen des brevets

(11) Publication number: **0 510 450 A1**

# EUROPEAN PATENT APPLICATION

(21) Application number: **92106149.5**

(22) Date of filing: **09.04.92**

(51) Int. Cl.5: **A61F 5/41**

(30) Priority: **25.04.91 YU 755/91**

(43) Date of publication of application:
**28.10.92 Bulletin 92/44**

(84) Designated Contracting States:
**AT BE CH DE DK ES FR GB GR IT LI LU MC NL PT SE**

(71) Applicant: **Matejevic, Nenad**
**Lole Ribara 37**
**Beograd(YU)**
Applicant: **Kaludjerovic, Nenad**
**Matice Srpske 40**
**Beograd(YU)**
Applicant: **Vujovic, Zarko**
**Bulevar Avnoj-a 16**
**Beograd(YU)**

(72) Inventor: **Matejevic, Nenad**
**Lole Ribara 37**
**Beograd(YU)**
Inventor: **Kaludjerovic, Nenad**
**Matice Srpske 40**
**Beograd(YU)**
Inventor: **Vujovic, Zarko**
**Bulevar Avnoj-a 16**
**Beograd(YU)**

(74) Representative: **Casalonga, Axel et al**
**BUREAU D.A. CASALONGA - JOSSE**
**Morassistrasse 8**
**W-8000 München 5(DE)**

(54) **Foldable apparatus for achieving and mantaining penis erection.**

(57) The invention refers to the foldable apparatus for achieving and maintaining of erection of penis, based on the principle of the reduced air pressure effect, which can be folded, so that such folded apparatus occupies less space. The apparatus consists of two transparent parts, the lower part 1 and the upper part 3, which can be hermeticly interconnected by the threaded ring 7 and the seal ring 8. In order to decrease the dimensions of the apparatus, the upper part 3 enters the lower part 1. On the upper part 3 there is the head of the apparatus 16 and on its upper side there are the valve 18 and the ribbed pipe 17, over which the elastic hose 19 of the vacuum pump 20 is pulled. The double lever 14, by which the elastic ring 22 is put to the base of the penis, anatomically is adjusted to the hand of the user, while the elastic ring 22 has the double effect in the moment when its ends disconnect to enable an undisturbed ejaculation.

FIG. 1

The invention refers to the foldable apparatus for achieving and maintaining penis erection, described in the first part of the Claim 1.

By this invention, the men's problem of achieving and maintaining of the penis erection has been solved based on the known principle of exposing the penis to the reduced air pressure and, owing to the construction of the apparatus which eliminates all the essential problems which were noticed in the previous such constructions and which appear in practice during the use of the apparatus.

Namely, all the apparatuses for this purpose, known until now, in our country and worldwide, have very big dimensions, which creates to the users great psycho-physical disturbances while carrying the apparatus and during its discrete use. Also, the apparatuses known until now, have unsuitable levers for displacing the elastic rings which tighten the penis, from the neck of the apparatus to the base of the penis. The known solutions, for this purpose use a double manual lever, which is hard to handle and which is not anatomically adjusted to the hand of the user while performing the said action. Also, all the known kinds of the elastic rings used for the tightening of penis until now, for the sake of the fast placing of their ends in the pre-ejaculation moment, use the system of pulling the small balls or thread in one strictly determined direction, which always creates a confusion to the user, in the very critical moment of the intercourse, since he is never quite sure in which of the two possible directions, in relation to the vertical axis of the penis, he should pull the small ball or the thread, because the direction depends on the direction in which he placed the ring to the apparatus.

The main characteristics of this foldable apparatus are:

1. The foldable construction enabling the user to carry the apparatus from one place to another, while the apparatus occupies very little space, since the parts may be packed one into another.

2. The use of the double lever, has solved the problem which appears during the handling with the previous levers for displacing the rings which tighten the base of the penis, from the neck of the apparatus to the base of the penis, provided the maximal anatomical adaptation to the hand of the user.

3. The construction of the new elastic ring with the elastic connection which enables the prompt disassembling, no matter in which position the elastic ring was put to the neck of the apparatus.

4. High quality and simple construction which, besides beeing functional, also provides a goods disign of the apparatus, which is of great importance during the use of the apparatus before the very intercourse, because it eliminates the physical traumas which the users have and which arise from the very fact that they have to use any aids in order to achieve and maintain the erection of the penis.

There is a lot of professional and patent documentation in the world, dealing with the solvation of the problem of penis erection.

For the sake of comparison, we herewith mention some of the disigns from the Europe and American Patent Office, with a brief view over their shortcomings:

- The German patent No. 268871 for the solution named: "Vorrichtung zur kunstlichen Erektion des Penis", which by its construction very much differs from the invention in this Claim. Naimely, according to the patent No. 268874, the apparatus consists of a simple tube with a ball-shape pump on the top and an additional ring on the other end and being such, it is not applicable for the various dimensions of penises.

- The European patent No. 734, 394 for the solution named: Improvements in or relating to Medical Appliances", relating to the adjustable rubber ring for tightening the base of the penis and, which construction completely differs from the rubber ring according to this Claim.

- The American patent No. 2, 874, 698 for the solution named: "Erector", which has some elements in the construction similar to the solution in this Claim, but they are full of faults. Namely, "Erector" has the central tube on which there is the puller with the lever for displacing of the ring and, the pump part on the very top, with a separate vacuum cylinder. But, the very construction of the puller with the lever, is bad, because it is not adjusted to the anatomy of the user and, also the lever can easily fall down from the puller during the procedure of displacing the ring and placing it to the base of the penis. Also, the upper part of the pump is relatively awkwardly constructed and placed so high that the user feels uncomfortable while pumping in order to achieve the erection of the penis.

- The American patent No. 3, 744, 486 for the solution named: "Apparatus for Obtaining an Artificial Erection", the construction of which absolutely differs from the solution in this Claim, because on the main tube, it has a special construction of the lever with the pump cylinder, which are very awkward and unpleasant for the user during the function of pumping in order to obtain the penis erection. The construction of the rubber ring, used for tightening the base of the penis, is also different from the solution of the rubber ring in this Claim and, the main fault is that it is very difficult to remove from the base of the penis.

- The British patent No. 1 497 441 for the solution named: "Appliance to Help Men Suffering from Sexual Impotence", construction of which differs from the solution in this Claim and, its biggest disadvantage is the special belt fastened in the area of abdomen.

- The American patent No. 4, 741, 329 for the solution named: "Surgical Appliance for Stimulating an Erection" construction of which cannot be compared to the solution against this Claim, since it is relatively complicated both in respect of its design and the numerous installed elements, which means that it can be damaged. Also, the problem of displacing resp., placing the rubber ring to the base of the penis, was not adequately solved.

- The American patent No. 4, 718, 411 for the solution named: "Male Erecting Device", which cannot be compared to the solution in this Claim, because of its relatively complicated construction.

- The American patent No. 4, 753, 227 for the solution named: "Erection Device and Method" which essentially differs from the solution in this Claim, not only regarding its construction, but also regarding the mode of displacing the rubber ring from the apparatus to the base of the penis.

- The American patent No. 4, 858, 4999 for the solution named: "Erection Device" which differs a lot from the constructional characteristics of the solution in this Claim, regarding its construction and the construction of the ring for tightening the base of the penis.

- The European patent claim No. 901141705 publication No. 0 417 438 Al for the solution named: "Apparatus For Achieving and Maintaining Penis Erection", which was the previous solution of one of the authors of the new solution in this Claim. Although there are some common construction details, this apparatus nevertleless differs a lot from the solution in this Claim, which consists of two parts and a separate vacuum pump and, also a better solution for the rubber ring for tightening the base of penis, which now has two heads for pulling the ends of the ring and releasing the base of the penis from the pressure, enabling the ejaculation.

The invention will be described in detail, by way of example, with reference to the accompanying drawings in which:

Fig. 1  Shows the apparatus in the condition ready to operate:

Fig. 2  Shows the apparatus in the condition ready to operate, but from the side where the lever for displacing of rubber ring is:

Fig. 3  Shows the apparatus folded, ready for storage or transportation;

Fig. 4  Shows one of the possible connections between the upper and lower part of the tube of the apparatus, by a square section seal;

Fig. 5  Shows one of the possible connections between the upper and the lower part of the tube of the apparatus, by a round section seal;

Fig. 6  Shows one of the possible connections between the upper and the lower part of the tube of the apparatus, by a triangle section seal;

Fig. 7  Shows three projections of the elastic rubber ring the ends of which are tied with an elastic clutch;

Fig. 8  Shows the shape of the elastic clutch, and,

Fig. 9  Shows the elastic rubber ring in the streched condition.

As it can be seen from the enclosed drafts, the bases of the apparatus is the tube which has its lower part 1 and the upper part 3. The diameter of the upper part 3 corresponds to the diameter of the hollow 2 of the lower part 1, in which it can penetrate to the notch 6. On the bottom of the upper part 3, there is a threaded ring 7 by which the upper part 3 is fixed to the lower part 1 in the way that the threaded ring 7 pushes the notch 6 which helps the upper part 3 to enter the hollow 2 of the lower part 1. Below the notch 6 there is the sealing ring 8 which leans on the upper edge of the lower part 1. The sealing ring 8 is placed in order to provide a hermetic interconnection of the lower part 1 and the upper part 3, whose hollows 2 and 5 then make a unique inner hollow in which the penis is inserted. The upper part 3, by its upper side and over the arch curve 4, ends by the head of the apparatus 16, on the upper surface of which there are valve 18 and the ribbed pipe 17. Onto the ribbed pipe, the elastic hose 19 of the rubber vacuum pump 20 is put, while the pump creates the necessary subpressure inside the apparatus which is made of two hollows 2 and 5. The vacuum pump 20 has on its bottom an air outlet 21. The lower part 1 continues to a cylindric entrance part 10 on which there is a ring bushing 9, by which the elastic ring 22 is displaced from the entrance part 10 where it was placed before using the apparatus. In the opening of the entrance part 10, a special ribbed rubber ring 11 is installed, providing the hermetic closing between the penis of the user and the apparatus, which is extremely important for the procedure of pumping to make certain subpressure inside the apparatus and thus to achieve the penis erection. On one side of the lower part 1, near the interconnection between the lower part 1 and the entrance part 10, there are two semicircular outlets 12, on which the two arms double lever 14 is rotationally put, on the axle 15. The lever 14 has two shorter arms 13 which lean onto the bushing 9, by their two sides of the lower part 1 and in the centre. The semicircular outlets 12 at the same time are the notch for the upper position of the bushing 9 when the apparatus is in use.

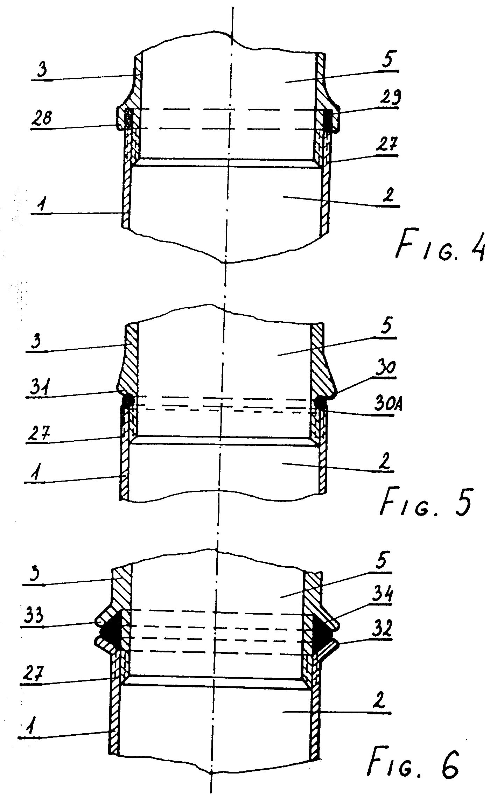

Since it is possible to use various types of sealing rings, the Fig. 4, 5 and 6 show various possible connections of the upper part 3 and the lower part 1 of the tube of apparatus. Figure 4 shows the connection of the parts of the tubus, with a sealing ring 29 with a square section. In order to provide a hermetic closing, the sealing ring 29 is tightened by a direct tightening of the threaded connections 27 of the upper part 3 into the lower part 1, whereby the sealing ring 29 is firmly inserted into the space made by the upper part 3 and the specially disigned ring 28. Figure 5 shows the connection of the parts of the tube, by a sealing ring 30, with a round section. The hermetic closing if provided by tightening the sealing ring 30 by the way of tightening the threaded connections 27 of the upper part 3 into the lower part 1, so that the sealing ring is firmly attached between the cone surface 30A of the lower part 1 and the ring 31 of the upper part 3. Figure 6 shows the connection with the already said characteristics, but with the sealing ring 34 of a triangular section, which is tightened between the cone surfaces 32 of the lower part 1 and the cone surfaces 33 of the upper part 3, in order to provide the hermetic closing.

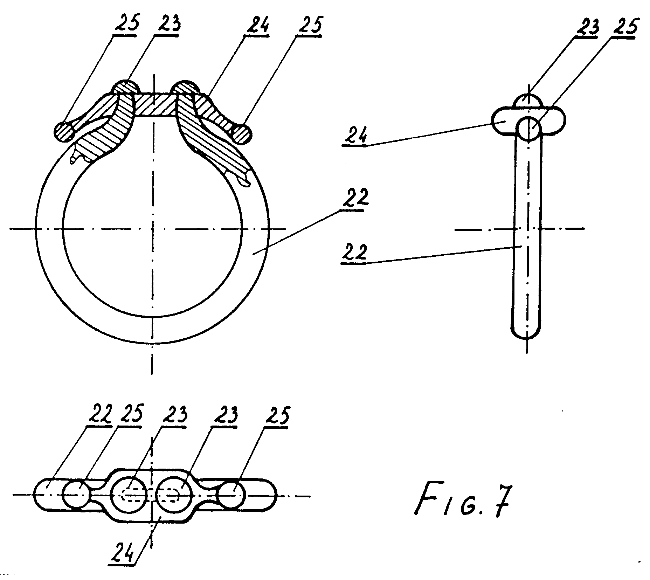

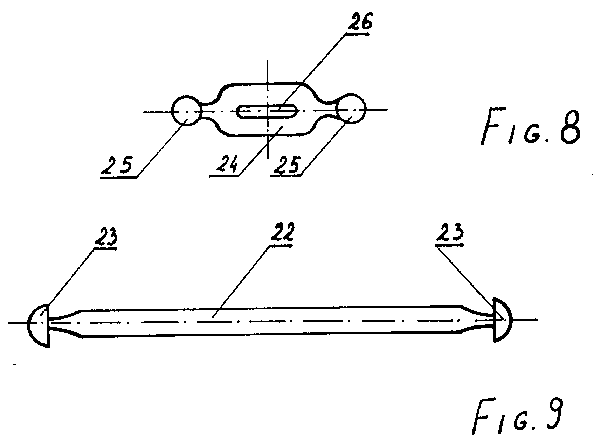

The problems which appear with the already knows solutions of the elastic rings which tighten the base of the penis, are solved by the construction of the new elastic ring 22 with the elastic connection 24, showed on Fig. 7, 8 and 9. The connection of the ends of this elastic ring 22 is done by the elastic connection 24 which has a slot 26 in the middle and in which both semicircular heads 23 of the elastic ring 22 are put, thus making an elastic connected ring. Since the elastic connection 24 of the elastic ring 22 has small balls 25 on both its ends, it means that when pulling any of these balls, the ends of the elastic ring 22 will immediately disconnect, no matter in which position the elastic ring 22 was put to the entrance part 10 of the apparatus for achieving and maintaining the erection of penis.

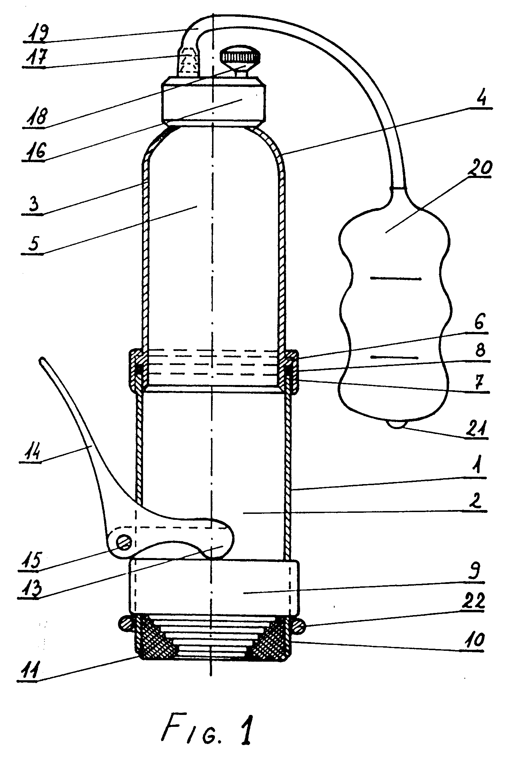

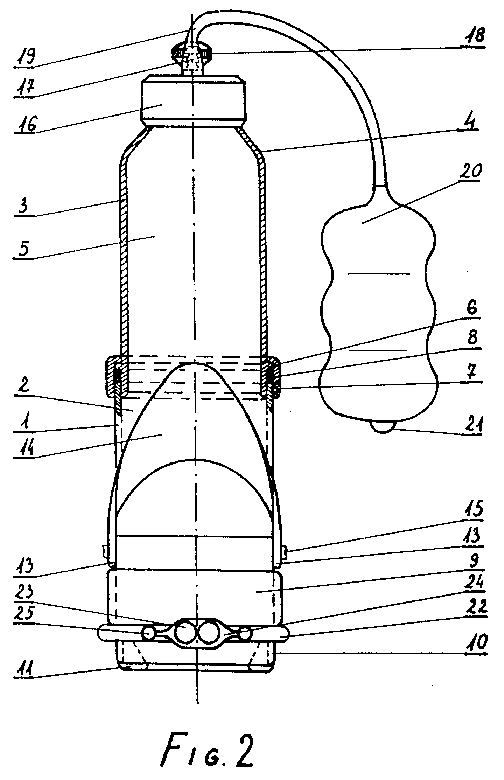

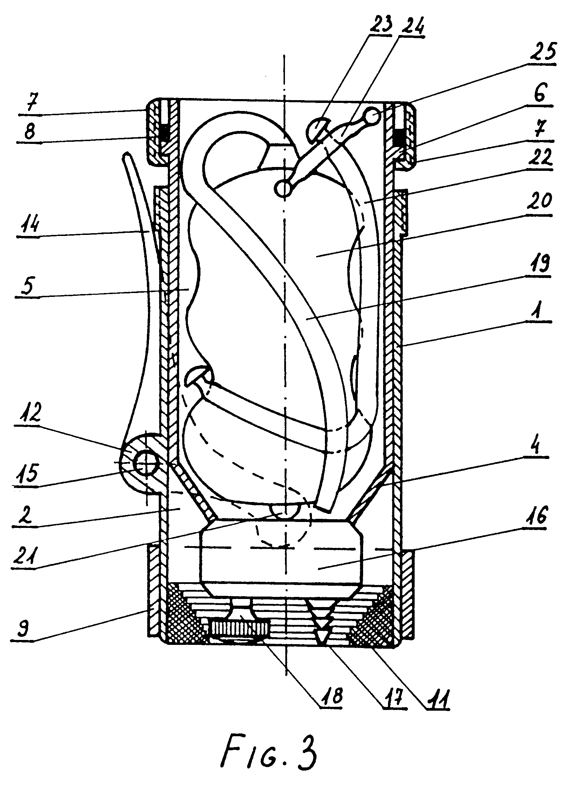

This apparatus for achieving and maintaining the erection of penis functions on the well known principle of exposing the penis to the reduced air pressure, produced by a vaccum pump 20. The apparatus can be carried, transported or kept, in the folded condition shown on Fig. 3. When needed, the parts of the apparatus are disassembled and one can form a normal apparatus shown on Fig. 1 and 2. In order to achieve the erection of penis, the following should be done:

- Through the opening of the ribbed rubber ring 11 at the entrance part 10, the penis is inserted into the inner hollow of the tube of the apparatus
- By a periodical pressing of the elastic vacuum pump 20, the prescribed subpressure is achieved in the tube of the apparatus, and the pressure is strictly limited by the construction of the vaccum pump 20
- After some time, depending on the previous sexual excitement of the user, the erection of the penis will occur. In order to maintain this erection even after taking the penis from the apparatus, the user should by any of his hands, grasp and press at the same time the longer arm of the lever 14 and the lower part 1 of the tube. The lever 14 moves round its axle 15 until its longer arm leans on the threaded ring 7. At the same time the shorter arm 13 of the lever 14 shall push forward the bushing 9. Bushing 9 shall move forward the elastic ring 22 until it slides down from the neck, i.e. from the entrance part 10 of the apparatus and tightens the base of the penis. In order to take the penis out of the tube of the apparatus after the described action, it is necessary to untighten a little, the valve 14 which is on the upper part of the head of the apparatus 16, then the air from outside will enter the tube and equilize with the air inside and, the penis may be taken out from the apparatus, in the condition of erection, after which the user can start the intercourse which will last 30 minutes of active sex.

At the end of the intercourse, just before the ejaculation, the user should, without taking penis from the vagina, pull any of the small balls 25 on the elastic connection 24 of the elastic ring 22, after which the ends of the elastic ring 22 will fastly disconnect and the semen will be normally expelled into the vagina.

## Claims

1. Foldable apparatus for achieving and maintaining erection of penis, based on the principle of the effect of the reduced air pressure to the outer surface of the penis;
   characterized by two parts, the lower part (1) and the upper part (3), where the disassembled upper part (3) can be put into the hollow (2) of the lower part (1) of the apparatus, in order to decrease the dimensions of the apparatus.

2. Apparatus according to Claim 1
**Characterized** by the upper part (3) which is connected to the lower part (1) by a threaded ring (7), while the hermetic closing is made by a seal ring (8).

3. Apparatus according to Claims 1 and 2
**Characterized** by the upper part (3) with the head of the apparatus (16) on the upper end of which there are valves (18) and a ribbed pipe (17) over which an elastic hose (19) of the vacuum pump (20) is put.

4. Apparatus according to Claim 2
**Characterized** by the upper part (3) connected to the lower part (1) by the threaded connection (27), while a seal ring (29) of the square section is inserted between the ring (28) of the upper part (3) and the edge of the wall of the lower part (1).

5. Apparatus according to Claim 2
**Characterized** by the round section seal ring (30) put between the ring (31) of the upper part (3) and the cone surface (30A) of the lower part (1).

6. Apparatus according to Claim 2
**Characterized** by a cone ring (32) on the lower part (1), while on the upper part (3) there is a cone ring (33) and a seal ring (34) of triangualar section is put between the cone surfaces of the cone ring.

7. Apparatus according to Claims 1 to 3
**Characterized** by the bottom of the lower part (1) where the entrance part (10) is, on which there is a ring bushing (9) and in the inner hollow there is a ribbed rubber ring (11) providing the hermetic connection between the base of the penis of the user and the apparatus.

8. Apparatus according to any of the previous Claims
**Characterized** by the sliding of the bushing (9),which enables the elastic ring (22) to slide from the entrance part (10) so that the base of the penis can be inserted; which sliding is done with the help of the double lever (14) which pushes forwards with its shorter arms (13) the bushing (9), when the strength of the hand moves the longer arm of the lever (14) in the direction of the tube of the apparatus.

9. Apparatus according to any of the previous Claims
**Characterized** by an elastic ring (22) used for tightening of the penis in order to maintain the erection, where the semicircular ends (23) are put in the slot (26) of the elastic connection (24) which has small balls (25) on its both ends and by pulling them the ends of the elastic ring (22) disconnect, thus enabling a natural ejaculation.

Fig. 1

**FIG. 2**

FIG.3

FIG. 4

FIG. 5

FIG. 6

FIG. 7

FIG. 8

FIG. 9

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| Y,D | EP-A-0 417 438 (MATEJEVIC ET AL.)<br>* the whole document *<br>--- | 1-4,7-9 | A61F5/41 |
| Y | EP-A-0 148 586 (NADIG ET AL.)<br>* page 4, line 16 - page 5, line 13; figures *<br>--- | 1-4,7-9 | |
| A,D | US-A-4 753 227 (YANUCK, JR.)<br>* column 4, line 32 - line 42; figures *<br>--- | 1 | |
| A,O | US-A-2 874 698 (SELL)<br>* column 1, line 41 - column 2, line 25; figures *<br>--- | 1 | |
| A | US-A-4 856 499 (KELLY)<br>--- | 1 | |
| X,P | US-A-5 020 522 (STEWART)<br>* column 5, line 58 - column 6, line 13; figures 1-5 *<br>----- | 1,2,5 | |
| | | | **TECHNICAL FIELDS SEARCHED (Int. Cl.5)**<br><br>A61F |

The present search report has been drawn up for all claims

| Place of search | Date of completion of the search | Examiner |
|---|---|---|
| THE HAGUE | 12 JUNE 1992 | SANCHEZ Y SANCHEZ J. |